(19) Europäisches Patentamt
European Patent Office
Office européen des brevets

(11) Veröffentlichungsnummer: **0 668 327 A1**

# (12) EUROPÄISCHE PATENTANMELDUNG

(21) Anmeldenummer: **95101053.7**

(51) Int. Cl.[6]: **C09B 62/085**

(22) Anmeldetag: **26.01.95**

(30) Priorität: **11.02.94 DE 4404341**

(43) Veröffentlichungstag der Anmeldung:
**23.08.95 Patentblatt 95/34**

(84) Benannte Vertragsstaaten:
**BE CH DE FR GB IE IT LI NL**

(71) Anmelder: **HOECHST AKTIENGESELLSCHAFT**
**Brüningstrasse 50**
**D-65929 Frankfurt am Main (DE)**

(72) Erfinder: **Russ, Werner Hubert, Dr.**
**Wingertstrasse 8a**
**D-65439 Flörsheim (DE)**
Erfinder: **Hussong, Kurt, Dr.**
**Gluckstrasse 19**
**D-65812 Bad Soden (DE)**

## (54) Wasserlösliche Azoverbindungen, Verfahren zu deren Herstellung und ihre Verwendung als Farbstoffe.

(57) Es werden Monoazoverbindungen beschrieben, die Farbstoffeigenschaften besitzen und hydroxy- und/oder carbonamidgruppenhaltiges Material, insbesondere Fasermaterial, wie Cellulosefasern, beispielsweise Baumwolle, Wolle und Nylon, in farbstarken, echten Tönen zu färben vermögen. Diese Monoazoverbindungen besitzen als Diazokomponente das 1-Sulfo-6-carboxy-2-amino-naphthalin und eine in der Farbstoffchemie übliche aminogruppenhaltige Kupplungskomponente, an deren Aminogruppe als faserreaktiver Rest ein 2-Halogen-s-triazin-6-yl-Rest gebunden ist, dessen 4-Stellung durch den Rest eines N-Heterocyclus oder durch eine Aminogruppe substituiert ist, die durch gegebenenfalls substituierte Alkyl- und Phenylreste substituiert sein kann.

EP 0 668 327 A1

Die Erfindung liegt auf dem technischen Gebiet der faserreaktiven Azofarbstoffe.

Faserreaktive Azofarbstoffe mit 2-Amino-naphthalin-disulfonsäuren als Diazokomponente sind zahlreich bekannt, beispielsweise aus den japanischen Patentanmeldungs-Veröffentlichungen Sho-59-133 261 und Sho-60-23 453 und aus den europäischen Patentanmeldungs-Veröffentlichungen Nrs. 0 128 034, 0 239 847 und 0 292 955. Des weiteren sind aus der europäischen Patentanmeldungs-Veröffentlichung Nr. 0 502 893 und den PCT-Patentanmeldungs-Veröffentlichungen WO 91/09913 (US-PS 5 268 458) und WO 91/09914 (US-PS 5 310 886) bereits faserreaktive Azofarbstoffe mit 1-Sulfo-6-carboxy-2-amino-naphthalin als Diazokomponente beschrieben. Die steigenden Anforderungen an die Qualität, die Wirtschaftlichkeit und die Brillanz der Färbungen haben es erforderlich gemacht, neue Azofarbstoffe zu entwickeln, die diesbezüglich verbesserte Eigenschaften besitzen und zudem anwendungstechnisch einfach zu handhaben sind.

Mit der vorliegenden Erfindung wurden nunmehr neue Azoverbindungen gefunden, die der allgemeinen Formel (1)

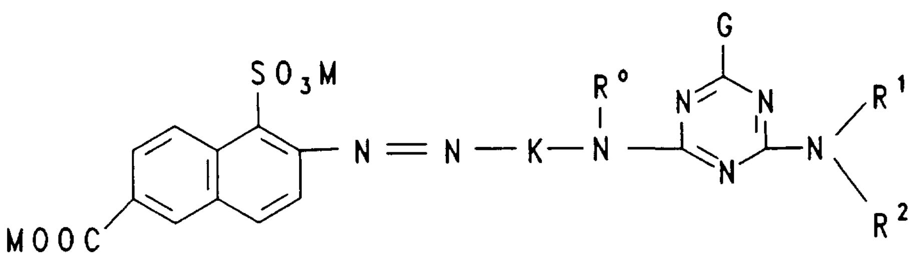

(1)

entsprechen, in welcher bedeuten:

- M ist Wasserstoff oder ein salzbildendes Metall, wie insbesondere ein Alkalimetall, wie Natrium, Kalium oder Lithium;
- K ist der bivalente, von der Gruppe der Formel -N($R^o$)- freie Rest einer wasserlöslichen aminogruppenhaltigen Kupplungskomponente;
- $R^o$ ist Wasserstoff oder Alkyl von 1 bis 4 C-Atomen, wie Methyl oder Ethyl, oder ist Alkyl von 1 bis 4 C-Atomen, wie beispielsweise Ethyl, das durch Sulfo, Carboxy, Sulfato, Phosphato, Hydroxy, Methoxy, Ethoxy, Phenyl, Monosulfophenyl oder Disulfophenyl substituiert ist, und ist bevorzugt Wasserstoff;
- G ist Halogen, wie Fluor oder Chlor, bevorzugt Fluor;
- $R^1$ ist Wasserstoff oder Alkyl von 1 bis 4 C-Atomen, wie Ethyl oder Methyl, oder ist Alkyl von 1 bis 4 C-Atomen, das durch Sulfo, Carboxy, Phosphato, Sulfato, Hydroxy, Cyano, Phenyl oder Sulfophenyl substituiert ist, oder ist Phenyl oder durch Sulfo, Carboxy, Alkyl von 1 bis 4 C-Atomen, Alkoxy von 1 bis 4 C-Atomen, Chlor und/oder Nitro substituiertes Phenyl;
- $R^2$ ist Wasserstoff oder Alkyl von 1 bis 4 C-Atomen, wie Ethyl oder Methyl, oder Alkyl von 1 bis 4 C-Atomen, das durch Sulfo, Carboxy, Phosphato, Sulfato, Hydroxy, Cyano, Phenyl oder Sulfophenyl substituiert ist, oder ist Phenyl, das durch 1, 2 oder 3 Substituenten substituiert sein kann, die aus der Gruppe der folgenden Substituenten ausgewählt sind: 2 Alkyl von 1 bis 4 C-Atomen, wie Ethyl und insbesondere Methyl, 2 Alkoxy von 1 bis 4 C-Atomen, wie Ethoxy und Methoxy, 1 Brom, 2 Chlor, 3 Sulfo, 2 Carboxy, 1 Nitro, 1 Alkylsulfonyl von 1 bis 4 C-Atomen, das durch Hydroxy substituiert sein kann (wie beispielsweise β-Hydroxyethylsulfonyl), 1 Alkylamino von 1 bis 4 C-Atomen und 1 Alkylamino von 1 bis 4 C-Atomen, das im Alkylrest durch Hydroxy, Sulfato, Sulfo, Phosphato, Alkanoyloxy von 2 bis 5 C-Atomen oder durch carboxysubstituiertes Alkanoylamido von 1 bis 4 C-Atomen im Alkylenrest, wie Succinamido, substituiert sein kann, oder $R^2$ ist Monosulfonaphthyl, Disulfonaphthyl oder Trisulfonaphthyl, oder
- $R^1$ und $R^2$ bilden zusammen mit dem Stickstoffatom einen aus einem Alkylen von 3 bis 8 C-Atomen, bevorzugt 4 bis 6 C-Atomen, oder einer weiteren Heterogruppe, wie einer Gruppe -NH- oder -O-, und zwei Alkylen von 1 bis 4 C-Atomen aufgebauten heterocyclischen Rest, wie beispielsweise die Gruppe N-Piperidino, N-Piperazino oder N-Morpholino.

Substituierte Alkylaminogruppen als Substituenten in einem Phenylrest von $R^2$ sind beispielsweise die β-Sulfatoethylamino- und die β-Succinamidoethyl-amino-Gruppe.

Bevorzugt ist der Rest -K-N($R^o$)- ein Rest der allgemeinen Formel (2a), (2b) oder (2c)

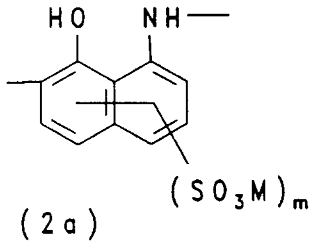

(2a)

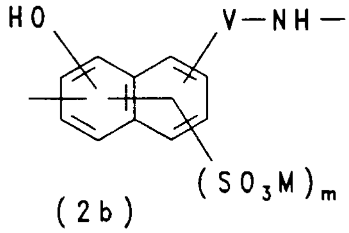

(2b)

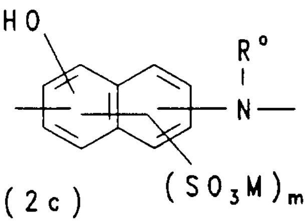

(2c)

in welchen

M und R° die obengenannten Bedeutungen haben,
m die Zahl 1 oder 2 bedeutet und
V eine direkte, kovalente Bindung oder ein Rest der Formel -NH-CO-phenylen-, -NH-CO-NH-phenylen-, $-N(CH_3)$-CO-phenylen-, $-N(CH_3)$-CO-NH-phenylen- oder -NH-phenylen- ist,

wobei in Formel (2a) im Falle von m gleich 2 die eine Sulfogruppe bevorzugt in meta-Stellung zur Hydroxygruppe und die andere Sulfogruppe bevorzugt in para- oder meta-Stellung zur Aminogruppe gebunden ist und in den Formeln (2b) und (2c) die freie Bindung, die zur Azogruppe führt, in ortho-Stellung zur Hydroxygruppe an den Naphthalinkern gebunden ist, die Hydroxygruppe bevorzugt in 8-Stellung an den Naphthalinkern gebunden ist, eine Sulfogruppe bevorzugt in 6-Stellung an den Naphthalinkern gebunden ist, die Gruppe -V-NH- bzw. -N(R°)- bevorzugt in 2- oder 3-Stellung an den Naphthalinkern gebunden ist und im Falle von m gleich 2 die Zweite Sulfogruppe bevorzugt in 3- und insbesondere bevorzugt in 4-Stellung an den Naphthalinkern gebunden ist.

Die vorstehend oder nachfolgend bezeichneten Gruppen "Sulfo", "Carboxy", "Sulfato" und "Phosphato" schließen sowohl deren Säureform als auch deren Salzform ein. Demgemäß bedeuten Sulfogruppen Gruppen entsprechend der allgemeinen Formel $-SO_3M$ , Carboxygruppen Gruppen entsprechend der allgemeinen Formel -COOM , Phosphatogruppen Gruppen entsprechend der allgemeinen Formel $-OPO_3M_2$ und Sulfatogruppen Gruppen entsprechend der allgemeinen Formel $-OSO_3M$ , jeweils mit M der obengenannten Bedeutung.

In den Formeln (2b) und (2c) stehen die Hydroxygruppe und die freie Bindung im selben aromatischen Kern in ortho-Stellung zueinander. Bevorzugt steht die Hydroxygruppe in α-Stellung an den Naphthalinrest gebunden.

Bevorzugt sind weiterhin Verbindungen der allgemeinen Formel (1), in welchen die Gruppe $-NR^1R^2$ ein oben definierter heterocyclischer Rest, bevorzugt hiervon N-Morpholino ist.

Bevorzugte Azoverbindungen der allgemeinen Formel (1) sind des weiteren solche, in denen der Rest -K-N(R°)- einen Rest der allgemeinen Formel (3a) oder (3b)

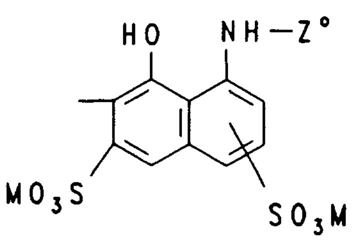

(3a)

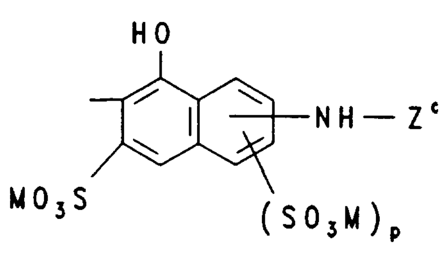

(3b)

bedeutet, in welchen

M die obengenannte Bedeutung hat,

Z° ein Rest der allgemeinen Formel (3A)

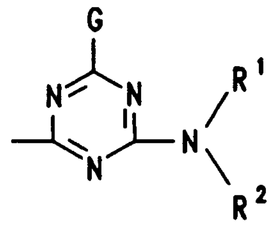

(3A)

mit G, $R^1$ und $R^2$ der obigen, insbesondere bevorzugten, Bedeutungen ist und

p die Zahl Null oder 1 ist (wobei im Falle p gleich Null diese Gruppe Wasserstoff bedeutet),

wobei in Formel (3a) die eine Sulfogruppe in meta- oder para-Stellung zur Gruppe -NH-Z° steht und in Formel (3b) die Gruppe -NH-Z° in 2-, 3- oder 4-Stellung, bevorzugt in 2- oder 3-Stellung, an den 8-Hydroxy-naphthalin-Rest gebunden ist und im Falle von p gleich 1 diese Sulfogruppe in 3- oder 4-Stellung, bevorzugt in 4-Stellung, an den 8-Hydroxy-naphthalin-Rest gebunden ist.

Insbesondere bevorzugt sind Verbindungen der allgemeinen Formeln (1A), (1B), (1C) und (1D)

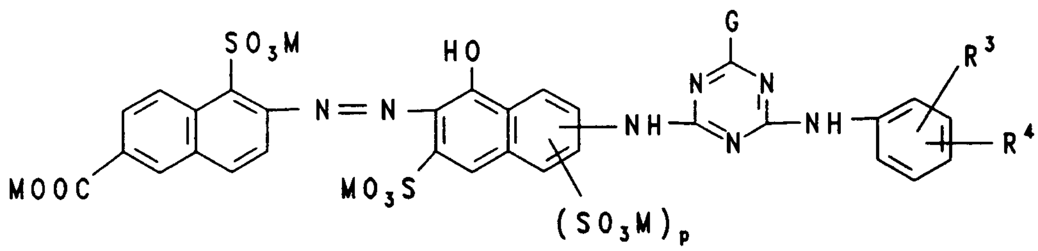

(1A)

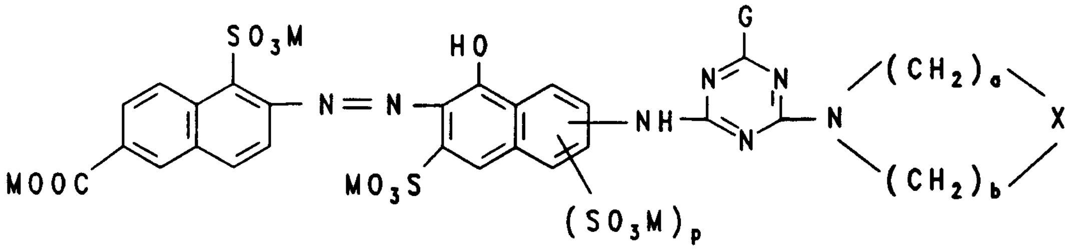

(1B)

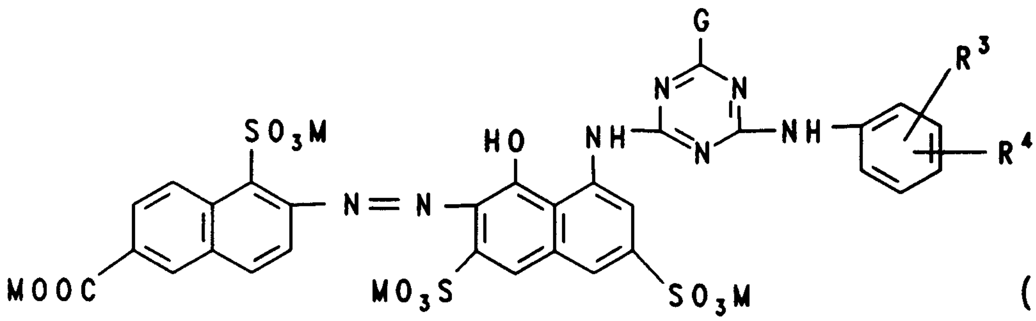

(1C)

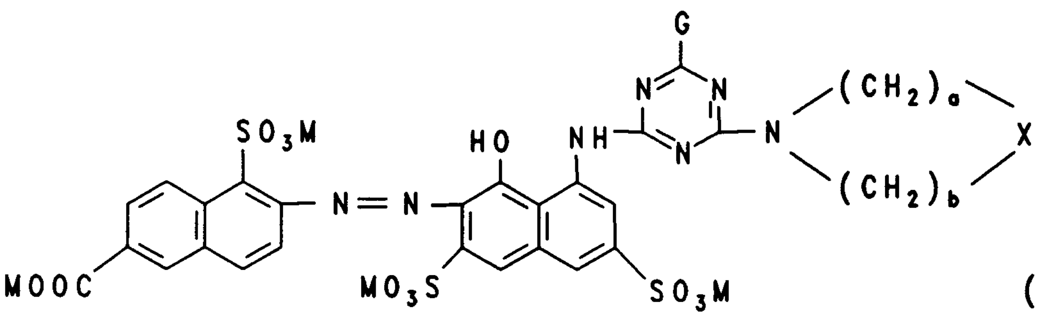

(1D)

in welchen

| | |
|---|---|
| M, G und p | die obengenannten, insbesondere bevorzugten Bedeutungen haben, |
| a | die Zahl 1, 2 oder 3, bevorzugt 2, ist, |
| b | die Zahl 1, 2 oder 3, bevorzugt 2, ist, |
| X | Sauerstoff oder die Gruppe -NH- ist, |
| $R^3$ | Wasserstoff, Sulfo, Carboxy, Alkyl von 1 bis 4 C-Atomen, wie Ethyl und insbesondere Methyl, Alkoxy von 1 bis 4 C-Atomen, wie Ethoxy und insbesondere Methoxy, oder Hydroxy ist und |
| $R^4$ | Wasserstoff, Sulfo, Carboxy, Alkyl von 1 bis 4 C-Atomen, wie Ethyl und insbesondere Methyl, oder Alkoxy von 1 bis 4 C-Atomen, wie Ethoxy und insbesondere Methoxy, ist, |

wobei in den Formeln (1A) und (1B) der Triazinylamino-Rest in 2- oder 3-Stellung an den 8-Hydroxy-naphthalin-Rest gebunden ist und im Falle von p gleich 2 diese Sulfogruppe in 3- oder 4-Stellung, bevorzugt in 4-Stellung, an den 8-Hydroxy-naphthalin-Rest gebunden ist.

Die vorliegende Erfindung betrifft weiterhin Verfahren zur Herstellung der erfindungsgemäßen Azoverbindungen der allgemeinen Formel (1), die dadurch gekennzeichnet sind, daß man die Diazoniumverbindung eines Amins der allgemeinen Formel (4)

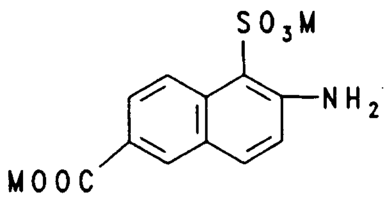

(4)

in welcher M die obengenannte Bedeutung hat, mit einer Verbindung der allgemeinen Formel (5)

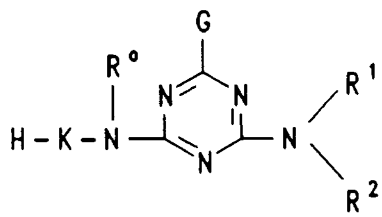

(5)

mit K, R°, G, $R^1$ und $R^2$ der obengenannten Bedeutung kuppelt, oder daß man eine Verbindung der allgemeinen Formel (6)

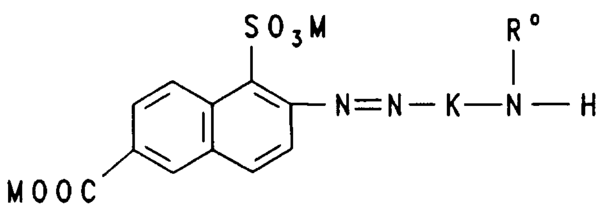

(6)

in welcher M, K und R° die obengenannten Bedeutungen haben, mit einer Verbindung der allgemeinen Formel (7)

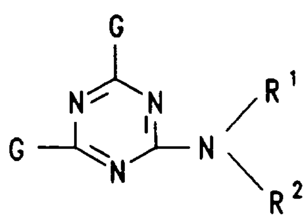

(7)

in welcher G, $R^1$ und $R^2$ die obengenannten Bedeutungen besitzen, umsetzt, oder daß man eine Verbindung der allgemeinen Formel (8)

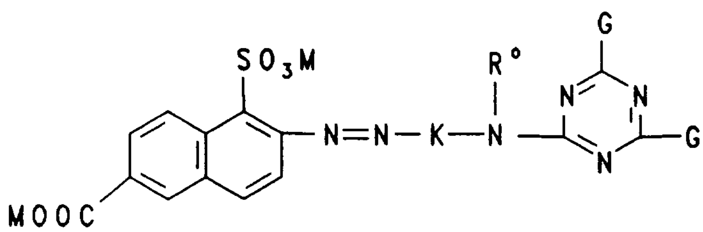

(8)

in welcher M, K, $R^o$ und G die obengenannten Bedeutungen besitzen, mit einer Aminoverbindung der allgemeinen Formel $HNR^1R^2$ mit $R^1$ und $R^2$ der obengenannten Bedeutung umsetzt.

Die Diazotierungs- und Kupplungsreaktionen des erfindungsgemäßen Verfahrens erfolgen in üblicher und altbekannter Weise, so die Diazotierung in der Regel bei einer Temperatur zwischen -5 °C und + 15 °C und einem pH-Wert unterhalb von 2 mittels einer starken Säure und Natriumnitrit in bevorzugt wäßrigem Medium und die Kupplungsreaktion in der Regel bei einer Temperatur zwischen 0 und 30 °C und bei einem pH-Wert zwischen 1 und 4,5 im Falle einer aminogruppenhaltigen Kupplungskomponente und bei einem pH-Wert zwischen 3 und 7,5 im Falle einer hydroxygruppenhaltigen Kupplungskomponente, bevorzugt in wäßrigem Medium.

Die Kondensationsreaktion einer Amino-Ausgangsverbindung der allgemeinen Formel (6) mit einer Verbindung der allgemeinen Formel (7) erfolgt in üblicher Weise der Umsetzung einer Aminoverbindung mit einer ein reaktives Halogenatom enthaltenden Triazinverbindung, so in organischem oder bevorzugt wäßrig-organischem Medium, insbesondere bevorzugt in wäßrigem Medium, unter Zusatz eines säurebindenden Mittels, wie eines Alkali- oder Erdalkalicarbonats, Alkali- oder Erdalkalihydrogencarbonats oder -hydroxids oder Alkaliacetats, wobei die Alkali- und Erdalkalimetalle vorzugsweise Natrium, Kalium oder Calcium sind, oder eines tertiären Amins, wie beispielsweise Pyridin, Triethylamin oder Chinolin. Sofern diese Kondensationsreaktionen in organischem oder wäßrig-organischem Medium erfolgen, ist das (anteilige) organische Lösemittel Aceton, Dioxan oder Dimethylformamid. Insbesondere erfolgt diese Kondensationsreaktion bei einer Temperatur zwischen -10 °C und + 60 °C, vorzugsweise zwischen 30 und 45 °C, und einem pH-Wert zwischen 2 und 7, bevorzugt zwischen 2 und 4,5. Die Umsetzung mit einer Verbindung (7) mit G gleich einem Fluoratom erfolgt insbesondere bevorzugt bei einem pH-Wert zwischen 3 und 5 und bei einer Temperatur zwischen 0 °C und30 °C.

Die Kondensationsreaktion einer Halogeno-triazinylamino-Verbindung der allgemeinen Formel (8) mit einer Aminoverbindung entsprechend der allgemeinen Formel $HNR^1R^2$ erfolgt ebenfalls analog bekannten Verfahrensweisen solcher Halogeno-triazinylamino-Verbindungen mit Aminen, so auch in den oben angegebenen Reaktionsmedien unter Einsatz eines säurebindenden Mittels. In der Regel erfolgt diese Umsetzung bei einer Temperatur zwischen 0 °C und 80 °C und einem pH-Wert zwischen 2 und 8, wobei die Umsetzung mit Ausgangsverbindungen, in welchen Hal ein Chloratom bedeutet, bevorzugt bei einer Temperatur zwischen 50 und 70 °C und einem pH-Wert zwischen 2 und 4 und die Umsetzung mit Ausgangsverbindungen, in welchen Hal ein Fluoratom bedeutet, bevorzugt bei einer Temperatur zwischen 0 °C und 10 °C und einem pH-Wert zwischen 6 und 8 durchgeführt wird.

Ausgangsverbindungen der allgemeinen Formel $H\text{-}K\text{-}N(R^o)\text{-}H$ sind beispielsweise 1-Amino-3,6- oder -4,6-disulfo-8-naphthol, 7-Amino-3-sulfo-1-naphthol, 6-Amino-3-sulfo-1-naphthol, 6-Amino-3,5-disulfo-1-naphthol, 2-Amino-4-acetylamino-benzol-1-sulfonsäure, 2-Amino-8-naphthol-6-sulfonsäure, 6-Methylamino-3-sulfo-1-naphthol, 6-(4'-Aminophenyl)-amino-3-sulfo-1-naphthol und 1-Amino-2,4-disulfo-8-naphthol.

Ausgangsverbindungen entsprechend der allgemeinen Formel $HNR^1R^2$ sind beispielsweise 2-Sulfo-anilin, 3-Sulfo-anilin, 4-Sulfo-anilin, 2,4-Disulfo-anilin, 2,5-Disulfo-anilin, 5-Sulfo-2-methoxy-anilin, 4-Sulfo-2-methoxy-anilin, 5-Sulfo-2-methyl-anilin, 4-Sulfo-2-methyl-anilin, 3-Sulfo-4-methoxy-anilin, 4-Sulfo-2-methoxy-5-methyl-anilin, 4-Sulfo-2,5-dimethoxy-anilin, 5-Sulfo-2,4-dimethoxy-anilin, 4-Sulfo-5-methoxy-2-methyl-anilin, 5-Sulfo-2-chlor-anilin, 3-Sulfo-4-chlor-2-methyl-anilin, 4-Sulfo-5-chlor-2-methoxy-anilin, 5- oder 6-Sulfo-2-amino-naphthalin, 6,8-Disulfo-2-amino-naphthalin, 3,6,8-Trisulfo-2-amino-naphthalin, 8-Sulfo-2-amino-naphthalin, 4,6,8-Trisulfo-2-amino-naphthalin, 1,5- oder 1,6-Disulfo-2-amino-naphthalin, 4,8-Disulfo-2-amino-naphthalin, 6,7-Disulfo-2-amino-naphthalin, 5,7-Disulfo-2-amino-naphthalin, 5- oder 6-Sulfo-1-amino-naphthalin, 7-Sulfo-1-amino-naphthalin, 1-Sulfo-2-amino-naphthalin, 3,6- oder 4,6- oder 4,8-Disulfo-1-amino-naphthalin, β-Sulfo-ethylamin, β-Hydroxyethylamin, N-Methyl-N-(β-sulfatoethyl)-amin, N-Methyl-N-ethyl-amin, Dimethylamin, Diethylamin, β-Carboxyethylamin, 4-Sulfobenzylamin, 4-Sulfopenethyl-amin, Benzylamin, 2-Methylanilin, Anilin, 4-Chlor-anilin, N-Ethyl-anilin, N-Methyl-anilin, Ammoniak, Morpholin, Piperidin und Piperazin.

Die Abscheidung und Isolierung der erfindungsgemäßen Verbindungen der allgemeinen Formel (1) - im nachfolgenden als Verbindungen (1) bezeichnet - aus den wäßrigen Syntheselösungen kann nach allgemein bekannten Methoden für wasserlösliche Verbindungen erfolgen, so beispielsweise durch Ausfällen aus dem Reaktionsmedium mittels eines Elekktrolyten, wie beispielsweise Natriumchlorid oder Kaliumchlorid, oder aber durch Eindampfen der Reaktionslösung selbst, beispielsweise durch Sprühtrocknung.

Falls die letztgenannte Art der Isolierung gewählt wird, empfiehlt es sich vielfach, vor dem Eindampfen eventuell in den Lösungen vorhandene Sulfatmengen durch Fällung als Calciumsulfat und Abtrennung durch Filtration zu entfernen.

Die Verbindungen (1) haben faserreaktive Eigenschaften und besitzen sehr wertvolle Farbstoffeigenschaften. Sie können deshalb zum Färben (einschließlich Bedrucken) von hydroxygruppenhaltigen und/oder carbonamidgruppenhaltigen Materialien verwendet werden. Hierzu können auch die bei der Synthese der

Verbindungen (1) anfallenden Lösungen, gegebenenfalls nach Zusatz einer Puffersubstanz und gegebenenfalls auch nach Konzentrierung, direkt als Flüssigpräparation der färberischen Verwendung zugeführt werden.

Gegenstand der vorliegenden Erfindung ist deshalb auch die Verwendung der Verbindungen (1) zum Färben (einschließlich Bedrucken) von hydroxy- und/oder carbonamidgruppenhaltigen Materialien bzw. Verfahren zu deren Anwendung auf diesen Substraten. Bevorzugt kommen die Materialien in Form von Fasermaterialien zur Anwendung, insbesondere in Form von Textilfasern, wie Garnen, beispielsweise in Form von Strängen oder Wickelkörpern, und Geweben. Hierbei kann man analog bekannten Verfahrensweisen vorgehen.

Hydroxygruppenhaltige Materialien sind solche natürlichen oder synthetischen Ursprungs, wie beispielsweise Cellulosefasermaterialien oder deren Regeneratprodukte und Polyvinylalkohole. Cellulosefasermaterialien sind vorzugsweise Baumwolle, aber auch deren Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane, insbesondere in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten, insbesondere auf den genannten Fasermaterialien, nach den für wasserlösliche, faserreaktive Farbstoffe bekannten Anwendungstechniken applizieren und fixieren, so beispielsweise, indem man die Verbindung (1) in gelöster Form auf das Substrat aufbringt oder sie darin einbringt und sie auf diesem oder in diesem durch Hitzeeinwirkung oder durch Einwirkung eines alkalisch wirkenden Mittels oder durch beide Maßnahmen fixiert. Solche Färbe- und Fixierweisen sind in der Literatur zahlreich beschrieben (beispielsweise in der europäischen Patentanmeldungs-VeröffentlichungNr. 0 181 585). Die Verbindungen (1) zeichnen sich durch hohe Auszieh- und Fixiergrade aus. Insbesondere in den Ausziehverfahren liefern sie, auch in Temperaturbereichen (40 bis 80 °C) Färbungen mit hoher Farbstärke bei hohem Fixiergrad.

Die erfindungsgemäßen Färbungen besitzen, insbesondere auf Cellulosefasermaterialien, gute Lichtechtheiten sowohl im trockenen Zustand der Färbung als auch im nassen, beispielsweise mit einer Schweißlösung befeuchteten, Zustand sowie gute Naßechtheiten, wie beispielsweise gute Waschechtheiten bei 60 bis 95 °C, auch in Gegenwart von Perboraten, saure und alkalische Walk-, Überfärbe- und Schweißechtheiten, gute saure und alkalische Schweißechtheiten, eine hohe Dampfbeständigkeit, gute Säure-, Wasser- und Seewasserechtheiten, desweiteren eine gute Plissierechtheit, Bügelechtheit und Reibechtheit. Ebenso besitzen sie eine gute Säurelagerbeständigkeit ("acid fading") beim Lagern von feuchten, noch Essigsäure enthaltendem, gefärbtem Material.

Die nachstehenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in den Beispielen formelmäßig beschriebenen Verbindungen sind in Form der freien Säure angegeben; im allgemeinen werden sie in Form ihrer Alkalimetallsalze, wie Lithium-, Natrium- oder Kaliumsalze, hergestellt und isoliert und in Form ihrer Salze zum Färben verwendet.

Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Verbindungen angegebenen Absorptionsmaxima ($\lambda_{max}$) im sichtbaren Bereich wurden anhand ihrer Alkalimetallsalze in wäßriger Lösung ermittelt. In den Tabellenbeispielen sind die $\lambda_{max}$-Werte bei der Farbtonangabe in Klammern gesetzt; die Wellenlängenangabe bezieht sich auf nm.

Beispiel 1

31,9 Teile 1-Amino-3,6-disulfo-8-naphthol werden mit 16 Teilen Cyanurfluorid in 200 Teilen Wasser und 100 Teilen Eis bei einem pH-Wert von 7,5 und einer Temperatur zwischen 0 °C und 10 °C kondensiert. Danach gibt man eine Lösung von 8,7 Teilen Morpholin in 200 Teilen Wasser hinzu und führt die zweite Kondensationsreaktion bei einem pH-Wert von 8,5 durch. Anschließend gibt man eine auf üblichem Wege salzsauer mit Natriumnitrit diazotierte Lösung des Diazoniumsalzes aus 27 Teilen 1-Sulfo-6-carboxy-2-amino-naphthalin in etwa 450 Teilen Wasser zu der so erhaltenen Lösung der Kupplungskomponente, stellt einen pH-Wert von 5 bis 6 ein und führt die Kupplungsreaktion innerhalb dieses pH-Bereiches bei einer Temperatur von etwa 20 °C durch.

Die erfindungsgemäße Azoverbindung der Formel (in Form der freien Säure geschrieben)

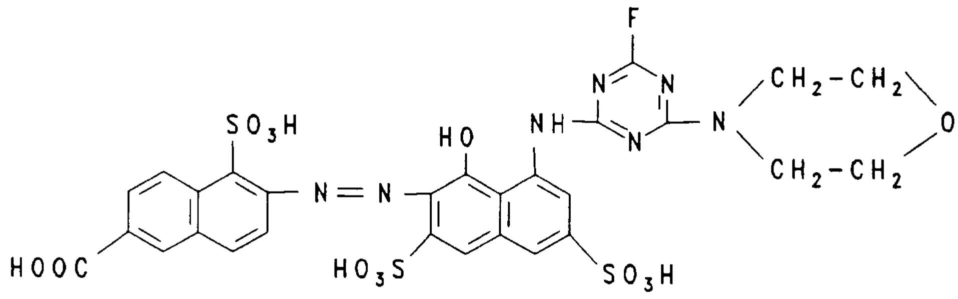

($\lambda_{max}$ = 545 nm)

wird mit Natriumchlorid ausgesalzen und als Natriumsalz isoliert. Sie besitzt sehr gute faserreaktive Farbstoffeigenschaften. Nach den in der Technik üblichen Anwendungsverfahren für faserreaktive Farbstoffe erhält man farbstarke Färbungen und Drucke in echten, blaustichig roten Tönen mit hoher Brillanz.

Beispiele 2 bis 25

Weitere erfindungsgemäße Farbstoffe entsprechend einer allgemeinen Formel (A)

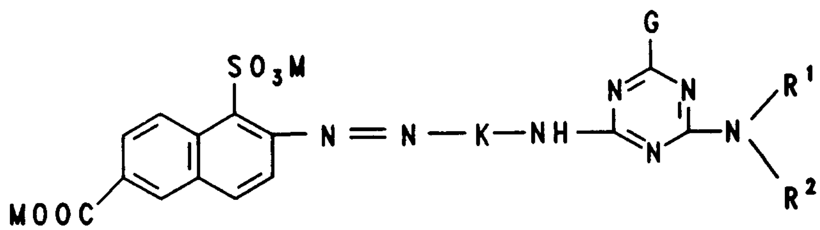

(A)

mit M der anfangs genannten Bedeutung sind in den nachfolgenden Tabellenbeispielen mit Hilfe der allgemeinen Formel (A) und der in der Tabelle angegebenen Formelreste beschrieben. Sie lassen sich in erfindungsgemäßer Weise, beispielsweise analog dem obigen Beispiel 1, herstellen und besitzen ebenfalls sehr gute faserreaktive Farbstoffeigenschaften. Nach den für faserreaktive Farbstoffe üblichen Applikations- und Fixierverfahren färben sie insbesondere Cellulosefasermaterialien in dem für das jeweilige Tabellenbeispiel angegebenen kräftigen echten Farbton.

| Bsp. | Rest -K-NH- | Rest G | Rest $-NR^1R^2$ | Farbton |
|---|---|---|---|---|
| 2 | 8-Hydroxy-3,6-disulfo-naphth-7-yl-1-ylamino | Fluor | Piperazino | rot (546) |
| 3 | dito | Fluor | Piperidino | rot (541) |
| 4 | dito | Fluor | 2-Methyl-phenylamino | rot (525) |
| 5 | dito | Fluor | 3-Sulfo-phenylamino | rot (515) |
| 6 | 8-Hydroxy-4,6-disulfo-naphth-7-yl-1-ylamino | Fluor | Phenylamino | rot (510) |

| Bsp. | Rest -K-NH- | Rest G | Rest $-NR^1R^2$ | Farbton |
|---|---|---|---|---|
| 7 | dito | Fluor | 3-Sulfo-phenylamino | rot |
| 8 | 8-Hydroxy-6-sulfo-naphth-7-yl-2-ylamino | Fluor | Morpholino | orange (483) |
| 9 | dito | Fluor | 3-Sulfo-phenylamino | orange (480) |
| 10 | dito | Fluor | N-Methyl-phenylamino | orange |
| 11 | 8-Hydroxy-6-sulfo-naphth-7-yl-3-ylamino | Fluor | Morpholino | scharlach (490) |
| 12 | dito | Fluor | 3-Sulfo-phenylamino | scharlach (492) |
| 13 | dito | Fluor | N-Methyl-phenylamino | scharlach (495) |
| 14 | dito | Fluor | N-Ethyl-phenylamino | scharlach (495) |
| 15 | 8-Hydroxy-4-sulfo-naphth-7-yl-1-ylamino | Fluor | 3-Sulfo-phenylamino | orange |
| 16 | dito | Chlor | dito | orange |
| 17 | 8-Hydroxy-6-sulfo-naphth-7-yl-3-ylamino | Chlor | 2,5-Disulfo-phenylamino | scharlach |
| 18 | dito | Chlor | 3-Sulfo-phenylamino | scharlach (493) |
| 19 | 8-Hydroxy-6-sulfo-naphth-7-yl-2-ylamino | Chlor | 2,5-Disulfo-phenylamino | orange |

| Bsp. | Rest -K-NH- | Rest G | Rest $-NR^1R^2$ | Farbton |
|---|---|---|---|---|
| 20 | 8-Hydroxy-3,6-disulfo-naphth-7-yl-1-ylamino | Chlor | N-Methyl-phenylamino | rot |
| 21 | dito | Chlor | 2-Methyl-phenylamino | rot |
| 22 | dito | Chlor | 3-Sulfo-phenylamino | rot (546) |
| 23 | 8-Hydroxy-4,6-disulfo-naphth-7-yl-1-ylamino | Chlor | N-Methyl-phenylamino | rot |
| 24 | dito | Chlor | 4-Sulfo-phenylamino | rot (520) |
| 25 | dito | Chlor | Morpholino | rot |

## Patentansprüche

**1.** Azoverbindung der allgemeinen Formel (1)

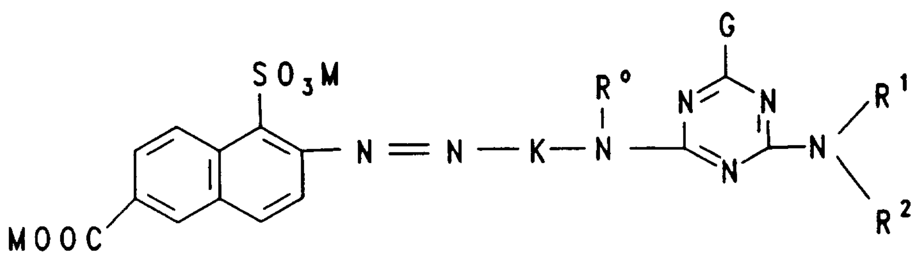

(1)

in welcher bedeuten:

- M ist Wasserstoff oder ein salzbildendes Metall;
- K ist der bivalente, von der Gruppe der Formel $-N(R^o)-$ freie Rest einer wasserlöslichen aminogruppenhaltigen Kupplungskomponente;
- $R^o$ ist Wasserstoff oder Alkyl von 1 bis 4 C-Atomen oder Alkyl von 1 bis 4 C-Atomen, das durch Sulfo, Carboxy, Sulfato, Phosphato, Hydroxy, Methoxy, Ethoxy, Phenyl, Monosulfophenyl oder Disulfophenyl substituiert ist;
- G ist Halogen;
- $R^1$ ist Wasserstoff oder Alkyl von 1 bis 4 C-Atomen oder ist Alkyl von 1 bis 4 C-Atomen, das durch Sulfo, Carboxy, Phosphato, Sulfato, Hydroxy, Cyano, Phenyl oder Sulfophenyl substituiert ist, oder ist Phenyl oder durch Sulfo, Carboxy, Alkyl von 1 bis 4 C-Atomen, Alkoxy von 1 bis 4 C-Atomen, Chlor und/oder Nitro substituiertes Phenyl;
- $R^2$ ist Wasserstoff oder Alkyl von 1 bis 4 C-Atomen oder Alkyl von 1 bis 4 C-Atomen, das durch Sulfo, Carboxy, Phosphato, Sulfato, Hydroxy, Cyano, Phenyl oder Sulfophenyl

substituiert ist, oder ist Phenyl, das durch 1, 2 oder 3 Substituenten substituiert sein kann, die aus der Gruppe der folgenden Substituenten ausgewählt sind: 2 Alkyl von 1 bis 4 C-Atomen, 2 Alkoxy von 1 bis 4 C-Atomen, 1 Brom, 2 Chlor, 3 Sulfo, 2 Carboxy, 1 Nitro, 1 Alkylsulfonyl von 1 bis 4 C-Atomen, das durch Hydroxy substituiert sein kann, 1 Alkylamino von 1 bis 4 C-Atomen und 1 Alkylamino von 1 bis 4 C-Atomen, das im Alkylrest durch Hydroxy, Sulfato, Sulfo, Phosphato, Alkanoyloxy von 2 bis 5 C-Atomen oder durch carboxysubstituiertes Alkanoylamido von 1 bis 4 C-Atomen im Alkylenrest substituiert sein kann, oder $R^2$ ist Monosulfonaphthyl, Disulfonaphthyl oder Trisulfonaphthyl, oder

$R^1$ und $R^2$ bilden zusammen mit dem Stickstoffatom einen aus einem Alkylen von 3 bis 8 C-Atomen oder einer weiteren Heterogruppe und zwei Alkylen von 1 bis 4 C-Atomen aufgebauten heterocyclischen Rest.

**2.** Azoverbindung nach Anspruch 1, dadurch gekennzeichnet, daß der Rest -K-N($R^o$)- ein Rest der allgemeinen Formel (2a), (2b) oder (2c)

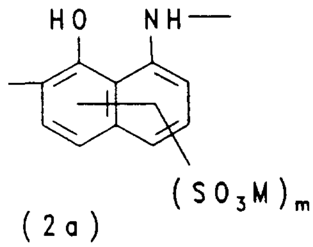
(2a)

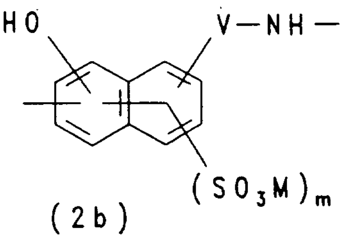
(2b)

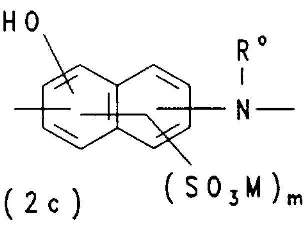
(2c)

ist, in welchen

M und $R^o$ die in Anspruch 1 genannten Bedeutungen haben,

m die Zahl 1 oder 2 bedeutet und

V eine direkte, kovalente Bindung oder ein Rest der Formel -NH-CO-phenylen-, -NH-CO-NH-phenylen-, -N($CH_3$)-CO-phenylen-, -N($CH_3$)-CO-NH-phenylen- oder -NH-phenylen- ist.

**3.** Azoverbindung nach Anspruch 1, dadurch gekennzeichnet, daß der Rest -K-N($R^o$)- ein Rest der allgemeinen Formel (3a) oder (3b)

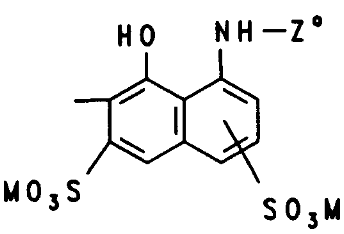
(3a)

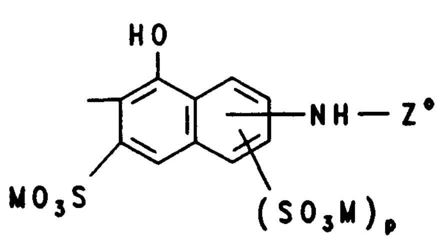
(3b)

ist, in welchen

M die in Anspruch 1 genannte Bedeutung hat,

Z° ein Rest der allgemeinen Formel (3A)

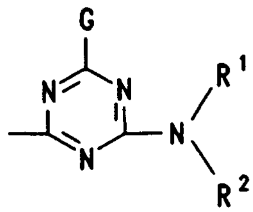

(3A)

mit G, $R^1$ und $R^2$ der in Anspruch 1 genannten Bedeutungen ist und

p die Zahl Null oder 1 ist (wobei im Falle p gleich Null diese Gruppe Wasserstoff bedeutet).

**4.** Azoverbindung nach Anspruch 1 entsprechend der allgemeinen Formel (1A)

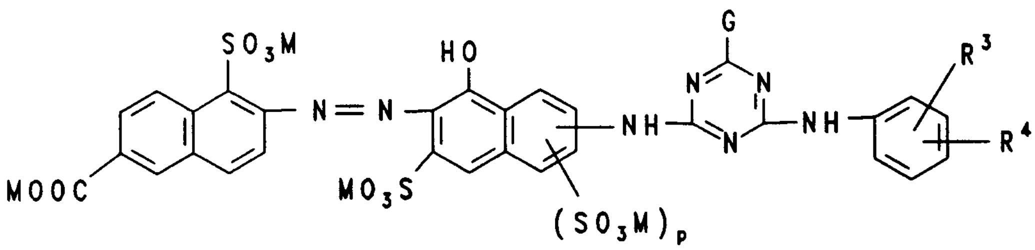

(1A)

in welchen

M und G die in Anspruch 1 genannten Bedeutungen haben,

p die Zahl Null oder 1 ist (wobei im Falle p gleich Null diese Gruppe Wasserstoff bedeutet),

$R^3$ Wasserstoff, Sulfo, Carboxy, Alkyl von 1 bis 4 C-Atomen, Alkoxy von 1 bis 4 C-Atomen oder Hydroxy ist und

$R^4$ Wasserstoff, Sulfo, Carboxy, Alkyl von 1 bis 4 C-Atomen oder Alkoxy von 1 bis 4 C-Atomen ist.

**5.** Azoverbindung nach Anspruch 1 der allgemeinen Formel (1B)

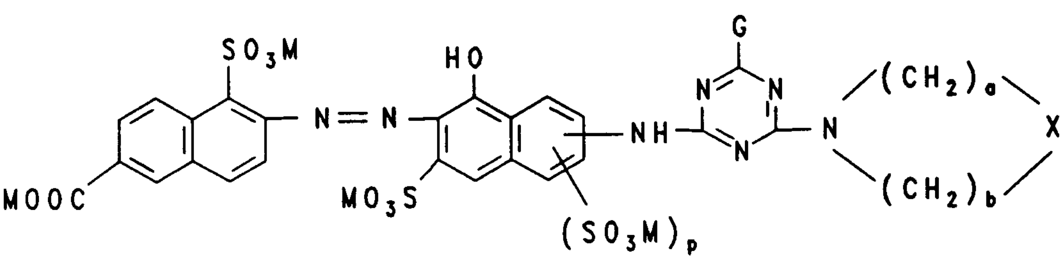

(1B)

in welcher

M und G die in Anspruch 1 genannten Bedeutungen haben,

p die Zahl Null oder 1 ist (wobei im Falle p gleich Null diese Gruppe Wasserstoff bedeutet),

a die Zahl 1, 2 oder 3 ist,

b die Zahl 1, 2 oder 3 ist und

X Sauerstoff oder die Gruppe -NH- ist.

**6.** Azoverbindung nach Anspruch 1 der allgemeinen Formel (1C)

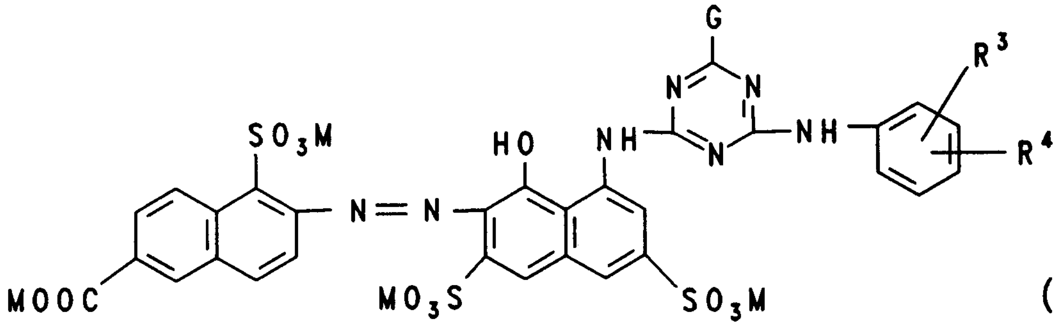

(1C)

in welcher M und G die in Anspruch 1 genannten Bedeutungen haben, $R^3$ Wasserstoff, Sulfo, Carboxy, Alkyl von 1 bis 4 C-Atomen, Alkoxy von 1 bis 4 C-Atomen oder Hydroxy ist und $R^4$ Wasserstoff, Sulfo, Carboxy, Alkyl von 1 bis 4 C-Atomen oder Alkoxy von 1 bis 4 C-Atomen ist.

**7.** Azoverbindung nach Anspruch 1 der allgemeinen Formel (1D)

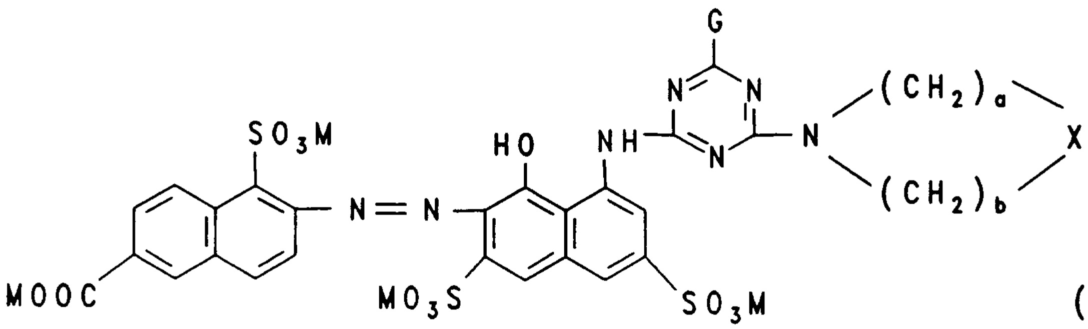

(1D)

in welcher M und G die in Anspruch 1 genannten Bedeutungen haben, $R^3$ Wasserstoff, Sulfo, Carboxy, Alkyl von 1 bis 4 C-Atomen, Alkoxy von 1 bis 4 C-Atomen oder Hydroxy ist und $R^4$ Wasserstoff, Sulfo, Carboxy, Alkyl von 1 bis 4 C-Atomen oder Alkoxy von 1 bis 4 C-Atomen ist.

**8.** Azoverbindung nach Anspruch 5 oder 7, dadurch gekennzeichnet, daß a die Zahl 2, b die Zahl 2 und X Sauerstoff ist.

**9.** Azoverbindung nach mindestens einem der Ansprüche 1 bis 3, dadurch gekennzeichnet, daß $R^o$ Wasserstoff ist.

**10.** Azoverbindung nach mindestens einem der Ansprüche 1 bis 9, dadurch gekennzeichnet, daß G Chlor ist.

**11.** Azoverbindung nach mindestens einem der Ansprüche 1 bis 9, dadurch gekennzeichnet, daß G Fluor ist.

**12.** Azoverbindung nach mindestens einem der Ansprüche 1 bis 3 und 9 bis 11, dadurch gekennzeichnet, daß die Gruppe $-NR^1R^2$ Morpholino ist.

**13.** Azoverbindung nach Anspruch 1 der Formel

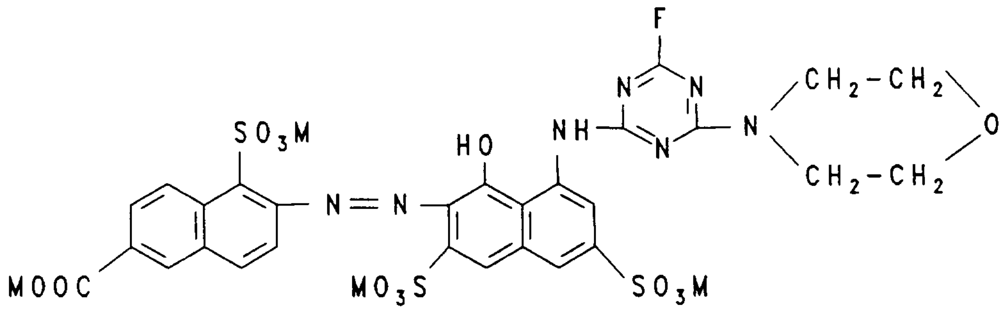

in welcher M die in Anspruch 1 genannte Bedeutung besitzt.

**14.** Verfahren zur Herstellung einer Azoverbindung der allgemeinen Formel (1) von Anspruch 1, dadurch gekennzeichnet daß man die Diazoniumverbindung eines Amins der allgemeinen Formel (4)

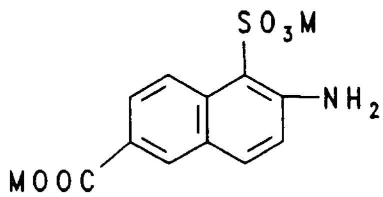

(4)

in welcher M die in Anspruch 1 genannte Bedeutung hat, mit einer Verbindung der allgemeinen Formel (5)

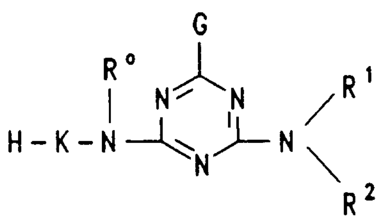

(5)

mit K, R°, G, $R^1$ und $R^2$ der in Anspruch 1 genannten Bedeutung kuppelt, oder daß man eine Verbindung der allgemeinen Formel (6)

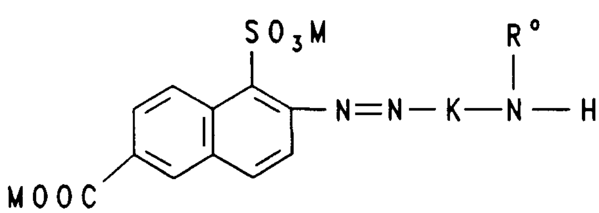

(6)

in welcher M, K und R° die in Anspruch 1 genannten Bedeutungen haben, mit einer Verbindung der allgemeinen Formel (7)

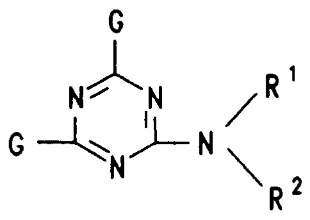

(7)

in welcher G, $R^1$ und $R^2$ die in Anspruch 1 genannten Bedeutungen besitzen, umsetzt, oder daß man eine Verbindung der allgemeinen Formel (8)

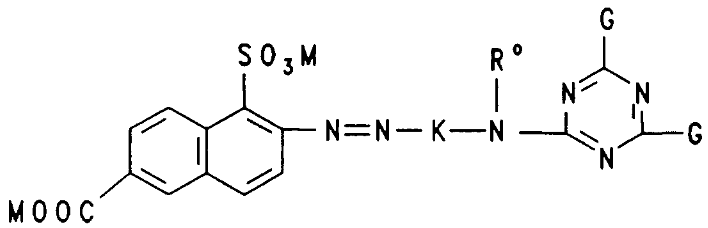

(8)

in welcher M, K, $R^o$ und G die in Anspruch 1 genannten Bedeutungen besitzen, mit einer Aminoverbindung der allgemeinen Formel $HNR^1R^2$ mit $R^1$ und $R^2$ der in Anspruch 1 genannten Bedeutung umsetzt.

**15.** Verwendung eines Farbstoffes von Anspruch 1 oder eines nach Anspruch 14 hergestellten Farbstoffes zum Färben (einschließlich Bedrucken) von hydroxy- und/oder carbonamidgruppenhaltigem Material, insbesondere Fasermaterial.

**16.** Verfahren zum Färben (einschließlich Bedrucken) von hydroxy- und/oder carbonamidgruppenhaltigem Material, insbesondere Fasermaterial, bei welchem man einen Farbstoff auf das Material aufbringt und den Farbstoff auf dem Material mittels Wärme oder mit Hilfe eines alkalisch wirkenden Mittels oder mittels beider Maßnahmen fixiert, dadurch gekennzeichnet, daß man als Farbstoff einen Farbstoff von Anspruch 1 oder einen nach Anspruch 14 hergestellten Farbstoff einsetzt.

Europäisches Patentamt

# EUROPÄISCHER RECHERCHENBERICHT

Nummer der Anmeldung
EP 95 10 1053

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| D,X | WO-A-91 09913 (HOECHST AG)<br>* Beispiele 17,21,24,28 *<br>* Beispiele 31,35 *<br>* Ansprüche 15-17 *<br>--- | 1,14-16 | C09B62/085 |
| A | CH-A-449 143 (CIBA AG)<br>* Spalte 1, Zeile 1 - Spalte 2, Zeile 34 *<br>--- | 1,14-16 | |
| A | GB-A-954 631 (ICI)<br>* Seite 5; Beispiel 16 *<br>--- | 1,14-16 | |
| A | EP-A-0 557 841 (BAYER AG)<br>* Seite 5, Zeile 10 - Zeile 28 *<br>----- | 5,7,13 | |

RECHERCHIERTE SACHGEBIETE (Int.Cl.6)

C09B

Der vorliegende Recherchenbericht wurde für alle Patentansprüche erstellt

| Recherchenort | Abschlußdatum der Recherche | Prüfer |
|---|---|---|
| DEN HAAG | 21.April 1995 | Ketterer, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)